Europäisches Patentamt

(19) European Patent Office

Office européen des brevets

(11) Numéro de publication: **0 004 211**
**B1**

(12) **FASCICULE DE BREVET EUROPEEN**

(21) Numéro de dépôt: **79400075.2**

(22) Date de dépôt: **06.02.79**

(51) Int. Cl.³: **C 07 C 79/18**

(54) **Préparation du nitro-2 méthyl-2 propanol-1**

(30) Priorité: **15.02.78 FR 7804210**

(43) Date de publication de la demande:
**19.09.79 Bulletin 79/19**

(45) Mention de la délivrance du brevet:
**10.12.80 Bulletin 80/25**

(84) Etats contractants désignés:
**BE CH DE FR GB IT LU NL SE**

(56) Documents cités:
**DE - B - 1 046 600**
**FR - A - 1 260 172**
**FR - A - 1 590 483**
**US - A - 2 139 120**
**US - A - 3 560 575**

(73) Titulaire: **SOCIETE CHIMIQUE DE LA GRANDE PAROISSE AZOTE ET PRODUITS CHIMIQUES**
**8, rue Coqnacq-Jay**
**F - 75007 Paris (FR)**

(72) Inventeur: **Adrian, Guy**
**9/12 Résidence Breteuil Parc Saint-Maur**
**F - 59000 Lille (FR)**
**Benattar, André**
**27, rue Jean Jaurès**
**F - 59000 Lille (FR)**
**Sion, Marcel-Xavier**
**450, Domaine du Chateau Lewarde**
**F - 59287 Lewarde (FR)**

(74) Mandataire: **Bouton Neuvy, Liliane, et al L'Air liquide, Société Anonyme pour l'Etude et L'Exploitation des Procédés Georges Claude**
**75, Quai d'Orsay**
**F - 75321 Paris Cedex 07 (FR)**

Courier Press, Leamington Spa, England.

Préparation du nitro-2 méthyl-2 propanol-1

La présente invention concerne la préparation du nitro-2 méthyl-2 propanol sous forme cristalline.

On connait par le brevet US 2.139.120 la synthèse de nitro-alcools par réaction d'une quantitié équimolaire ou d'un léger excès de nitroparaffine sur le formaldéhyde éventuellement sous forme solide en présence d'un hydroxyde de métal alcalino-terreux, selon lequel la neutralisation conduit à la formation d'un précipité de carbonate alcalino-terreux, éliminé par filtration, les nitro-alcools obtenus étant isolés par distillation sousvide et parfois purifiés par recristallisation.

Selon le brevet US 3560.575, on prépare en particulier la tris (hydroxyméthyl) nitrométhane (TN) en présence de soude avec désionisation du mélange réactionnel par fractionnement avec du méthanol en présence d'une résine échangeuse de cations.

La DAS 1.046.600 et le brevet français 1.260.712 ont trait à la préparation du β-nitro-éthanol. Le premier de ces procédés mis en oeuvre avec des quantités importantes de nitrométhane en présence d'hydroxyde de calcium en suspension exige une filtration et une distillation sous vide pour la récupération du β-nitroéthanol.

Selon le second procédé en milieu hydro-méthanolique, on est contrainte de procéder à un essorage, à une concentration, sous-vide puis à une distillation du nitrométhane en excès puis à une distillation sous-vide du β-nitro-éthanol.

On comprend que ce opérations de filtration, de concentration, d'extraction sélective et de cristallisation dans un solvant approprié soient longues et fastidieuses, nécessitant de plus le recyclage des solvants utilisés lors de la condensation et de la cristallisation, et aboutissent à des procédés peu éconimiques.

Il a été trouvé un procédé amélioré qui permet de supprimer les opérations de concentration, d'extraction sélective et en conséquence de recyclage des solvants, donc de pallier les inconvénients des procédés connus, et d'isoler le nitro-2 méthyl-2 propanol-1 avec une grande pureté directement du milieu réactionnel neutralisé, et avec des rendements pouvant être supérieurs à 95%.

Selon l'invention le nitro-2 propane et le formaldéhyde sont mis en réaction dans un rapport molaire compris entre 0,9:1 et 1,1:1, en milieu concentré et en présence de 1 à 10 milliéquivalents par mole de nitro-2 propane d'un catalyseur basique minéral, sous agitation pendant une durée de 1 à 48 heures, à température comprise entre 40 et 58°C; le pH du milieu réactionnel étant compris entre 7 et 11.

Le catalyseur basique minéral est avantageusement choisi parmi les hydroxydes alcalins, tel l'hydroxyde de potassium et l'hydroxyde de sodium. It peut être dissous dans l'eau.

Le maintien du pH du milieu réactionnel entre 8 et 9 conduit à des résultats très satisfaisants.

En général, le formaldéhyde est introduit en majeure partie sous forme de polyoxyméthylène.

Selon un mode de mise en oeuvre particulièrement intéressant les réactifs et le catalyseur sont respectivement mis en réaction dans l'ordre nitro-2 propane, formaldéhyde et catalyseur basique et en présence d'une quantité d'eau représentant 1,5 à 10% en poids de totalité de la masse réactionnelle. Cette faible quantité d'eua peut être introduite sous forme de solution aqueuse de formaldéhyde.

Le milieu réactionnel est ensuite neutralisé par un acide à température de l'ordre de 55°C. L'acide peut être un acide minéral ou organique tel l'acide chlorhydrique concentré et l'acide stéarique. L'agent neutralisant est ajouté en quantité telle que le pH du milieu réactionnel soit abaissé à un pH compris entre 4 et 5.

La cristallisation du nitro-2 méthyl-2 propanol est progressive par refroidissement jusqu'à la température ambiante ou agitation avec entrainement à l'aide d'un courant de gaz inerte de l'eua et des impuretés volatiles du milieu reactionnel brut. On obtient une fine poudre blanche de pureté supérieure à 95%, qui peut être mise en forme directement à l'aide d'une machine à pastiller ou d'extrudeuse.

Le nitro-2 méthyl-2 propanol a de nombreuses applications notamment comme agent d'adhésion dans la fabrication de pneumatiques, bactéricide et source de formaldéhyde en milieu basique.

Il est donné ci-après des exemples illustrant l'invention à titre non limitatif.

EXEMPLE 1

Dans un ballon agité pouvant être refroidi par immersion dans un bain d'eau froide, on introduit 90,5 g de nitro-2 propane (1 mole) auxquels on ajoute 8,7 g de solution aqueuse de formol à 30% (0,08 moles) 1,2 g de soude en solution à 400 g/litre (0,008 moles), et progressivement 29,8 g de polyoxyméthylène (0.97) moles) de façon que la température soit maintenue entre 45°C et 58°C. Après 1 heure de réaction à 50°C on neutralise à l'acide stéarique à 55°C jusqu'a pH = 4,3 et on laisse refroidir. Le produit est agité alors sous un courant d'air de façon à diviser les grumeaux de produit solide qui apparaissent. Après évaporation de la phase liquide, on obtient 112,6 g (0,94 moles) de nitro-2 méthyl-2 propanol en poudre de point de fusion 88°C. Le rendement est de 92% en un produit dont la microanalyse est correcte, la pureté est supérieure à 95%.

## EXEMPLE 2

On met en réaction dans une récipient agité refroidi par bain d'eau froide 136 g (1,5 mole) de nitro-2 propane auxquels on ajoute 13 g de solution aqueuse de formol à 30% (0,12 moles) et 1,5 g de soude à 400 g/litre (0,010 mole) et progressivement 45 g de polyoxyméthylène (1,42 mole). On laisse refroidir le produit et on l'envoie sur la vis de cristallisation définie à l'exemple n° 2. On obtient 163,2 g d'un produit de point de fusion 85°C rendement 92% d'un produit dont la microanalyse est bonne.

## EXEMPLE 3.

Dans le même récipient que dans l'exemple n° 3, on met en réaction 890 g (9,7 moles) de nitro-2 propane, 80 g solution aqueuse à 30% de formol (0,8 moles) et 15 g de solution de soude à 400 g/litre dans l'eau (0,1 mole) et on y ajoute progressivement 282,2 g de polyoxyméthylène (9,2 moles) en 1 heure. On neutralise 15' après la fin de l'introduction du polyoxy-méthylène par HCl concentré et on laisse refroidir à 45°C avant de l'envoyer sur la vis sans fin définie à l'exemple 2, on obtient 1 109 g d'un produit de point de fusion 86°C (rendement 96%) et dont la microanalyse correspond au nitro-2 méthyl-2 propanol-1.

## Revendications

1. Procédé de préparation directe de nitro-2 méthyl-2 propanol-1 cristallin du pureté supérieure à 95% par condensation du nitro-2 propane avec le formaldéhyde dans un rapport molaire compris entre 0,9—1,1:1 en présence de 1 à 10 milliéquivalents par mole de nitro-2 propane d'un catalyseur basique minéral, caractérisé en ce que le nitro-2 propane, la mineure partie du formaldéhyde sous forme liquide et le catalyseur basique sont mis en réaction dans l'ordre indiqué en présence d'une quantité d'eau représentant 1,5 à 10% de la totalité de la masse réactionelle, puis en ce qu'on ajoute progressivement sous agitation la majeure partie du formaldéhyde sous forme solide de polyoxyméthylène de manière à maintenir la température comprise entre 40 et 58°C; le pH du milieu réactionnel étant compris entre 7 et 11; après condensation le produit réactionnel formé est neutralisé à température de l'ordre de 55°C par un acide jusqu'à pH compris entre 4 et 5; et le dit produit réactionnel est obtenu sous forme cristalline par refroidissement ou agitation avec entrainement, à l'aide d'un courant de gaz inerte, de l'eau et des impuretés volatiles du milieu réactionnel brut.

2. Procédé de préparation du nitro-2 méthyl-2 propanol-1 cristallin selon la revendication 1, caractérisé en ce que le pH du milieu réactionnel est compris entre 8 et 9.

3. Procédé de préparation directe du nitro-2 méthyl-2 propanol-1 cristallin selon la revendication 1, caractérisé en ce que le catalyseur basique est l'hydroxyde de sodium ou l'hydroxyde de potassium.

4. Procédé de préparation directe du nitro-2 méthyl-2 propanol-1 cristallin selon la revendication 1, caractérisé en ce que la quantité contrôlée d'eau est introduite sous forme de solution aqueuse de formaldéhyde.

5. Procédé de préparation directe de nitro-2 méthyl-2 propanol-1 selon la revendication 1, caractérisé en ce que l'acide est l'acide stéarique.

6. Procédé de préparation directe de nitro-2 méthyl-2 propanol-1 selon la revendication 1, caractérisé en ce que l'acide est l'acide chlorhydrique.

7. Procédé de préparation directe de nitro-2 méthyl-2 propanol-1 cristallin de pureté supérieure à 95% par condensation du nitro-2 propane avec le formaldéhyde dans un rapport molaire compris entre 0,9—1,1:1 en présence de 1 à 10 milliéquivalents par mole de nitro-2 propane d'un catalyseur basique minéral, caractérisé en ce que le nitro-2 propane, la mineure partie du formaldéhyde sous forme liquide et le catalyseur basique hydroxyde de sodium ou de potassium sont mis en réaction dans l'ordre indiqué en présence d'une quantité d'eau représentant 1,5 à 10% de la totalité de la masse réactionnelle, puis en ce qu'on ajoute progressivement sous agitation la majeure partie du formaldéhyde sous forme solide de polyoxyméthylène de manière à maintenir la température comprise entre 40 et 58°C; le pH du milieu réactionnel étant compris entre 7 et 11; après condensation le produit réactionnel formé est neutralisé à température de l'ordre de 55°C par l'acide stéarique jusqu'à pH compris entre 4 et 5; et le dit produit réactionnel est obtenu sous forme cristalline par refroidissement ou agitation avec entrainement, à l'aide d'un courant de gaz inerte, de l'eau et des impuretés volatiles du milieu réactionnel brut.

## Claims

1. Process for the direct preparation of crystalline 2-nitro-2-methyl-propan-1-ol having a purity of more than 95% by condensation of 2-nitropropane with formaldehyde in a mol ratio of 0,9—1,1:1 in presence of 1 to 10 milli-equivalents per mol of 2-nitropropane of a basic mineral catalyst, characterized in that the 2-nitropropane, the minor proportion of the formaldehyde in liquid form and the basic catalyst are reacted in the stated order in presence of an amount of water representing 1,5 to 10% of the totality of the reaction mass, then progressively with agitation the major proportion of the formaldehyde in the solid form of polyoxymethylene is added, so that the temperature is maintained within the range of 40 to 58°C, the pH of the reaction medium being between 7 and 11, after condensation the formed product is neutralized at a temperature in the order of 55°C by an acid until a pH of between 4 and 5, and the said reaction product is obtained in crystalline form by cooling or agitation with entrainment of the water and the volatile impurities of the rough reaction medium by means of a stream of inert gas.

2. Process for the direct preparation of crystalline 2-nitro-2-methyl-propan -1-ol according to claim 1, characterized in that the pH of the reaction medium is between 8 and 9.

3. Process for the direct preparation of crystalline 2-nitro-2-methyl-propan -1-ol according to claim 1, characterized in that the basic catalyst is sodium hydroxide or potassium hydroxide.

4. Process for the direct preparation of crystalline 2-nitro-2-methyl-propan -1-ol according to claim 1, characterized in that the controlled amount of water is introduced in the form of an aqueous solution of formaldehyde.

5. Process for the direct preparation of 2-nitro-2-methyl-propan -1-ol according to claim 1, characterized in that the acid is stearic acid.

6. Process for the direct preparation of 2-nitro-2-methyl-propan -1-ol according to claim 1, characterized in that the acid is hydrochloric acid.

7. Process for the direct preparation of crystalline 2-nitro-2-methyl-propan -1-ol having a purity of more than 95% by condensation of 2-nitropropane with formaldehyde in a mol ratio of 0,1—1,0:1 in presence of 1 to 10 milliequivalents per mol of 2-nitropropane of a basic mineral catalyst, characterized in that the 2-nitropropane, the minor proportion of the formaldehyde in liquid form and the basic sodium or pottassium hydroxide catalyst are reacted in the stated order in presence of an amount of water representing 1,5 to 10% of the totality of the reaction mass, then progressively with agitation the major portion of the formaldehyde in the solid form of polyoxymethylane is added so that the temperature is maintained between 40 and 58°C, the pH of the reaction medium being between 7 and 11, after condensation the formed reaction product is neutralized at a temperature in the order of 55°C by means of stearic acid until a pH between 4 and 5, and the said reaction product is obtained in crystalline form by cooling or agitation with entrainment of the water and the volatile impurities of the rough reaction medium by means of a stream of inert gas.

**Patentansprüche**

1. Verfahren zur direkten Herstellung von kristallinem 2-Nitro-2-methyl-propan-1-ol einer Reinheit von mehr als 95% durch Kondensation von 2-Nitropropan mit Formaldehyd in einem Molverhältnis von 0,9 bis 1,1:1 in Gegenwart von 1—10 Milliäquivalenten eines basischen mineralischen Katalysators je Mol 2-Nitropropan, dadurch gekennziechnet, daß das 2-Nitropropan, der kleinere Teil des Formaldehyds in flüssiger Form und der basische Katalysator in der angegebenen Reihenfolge in Gegenwart einer Wassermenge entsprechend 1,5 bis 10% der Gesamtheit der Reaktionsmasse miteinander umgesetzt werden, sodann nach und nach unter Rühren der größbere Teil des Formaldehyds in fester Polyoxymethylenform derart zugegeben wird, daß die Temperatur zwischen 40 und 58°C gehalten wird, wobei der pH-Wert des Reaktionsmediums zwischen 7 und 11 liegt, nach der Kondensation das gebildete Reaktionsprodukt bei einer Temperatur in der Größenordnung von 55°C mit einer Säure bis zu einem pH-Wert zwischen 4 und 5 neutralisiert wird und dieses Reaktionsprodukt in kristalliner Form durch Abkühlen oder Rühren unter Mitreißen des Wassers und der flüchtigen Verunreinigungen des rohen Reaktionsmediums mit Hilfe eines Inertgasstromes erhalten wird.

2. Verfahren zur direkten Herstellung von kristallinem 2-Nitro-2-methyl-propan-1-ol nach Anspruch 1, dadurch gekennzeichnet, daß der pH-Wert des Reaktionsmediums zwischen 8 und 9 liegt.

3. Verfahren zur direkten Herstellung von kristallinem 2-Nitro-2-methyl-propan-1-ol nach Anspruch 1, dadurch gekennzeichnet, daß der basische Katalysator Natriumhydroxid oder Kaliumhydroxid ist.

4. Verfahren zur direkten Herstellung von kristallinem 2-Nitro-2-methyl-propan-1-ol nach Anspruch 1, dadurch gekennzeichnet, daß die kontrollierte Menge an Wasser in der Form einer wäßrigen Formaldehydelösung eingeführt wird.

5. Verfahren zur direkten Herstellung von 2-Nitro-2-methyl-propan-1-ol nach Anspruch 1, dadurch gekerinzeichnet, daß die Säure Stearinsäure ist.

6. Verfahren zur direkten Herstellung von 2-Nitro-2-methyl-propan-1-ol nach Anspruch 1, dadurch gekennzeichnet, daß die Säure Chlorwasserstoffsäure ist.

7. Verfahren zur direkten Herstellung von kristallinem 2-Nitro-2-methyl-propan-1-ol einer Reinheit oberhalb 95% durch Kondensation von 2-Nitropropan mit Formaldehyd in einem Molverhältnis von 0,9 bis 1,1:1 in Gegenwart von 1 bis 10 Milliäquivalenten eines basischen mineralischen Katalysators je Mol 2-Nitropropan, dadurch gekennzeichnet, daß das 2-Nitropropan, der kleinere Teil des Formaldehyds in flüssiger Form und der basische Katalysator in der Form von Natrium- oder Kaliumhydroxid in der angegebenen Reihenfolge in Gegenwart einer Wassermenge entsprechend 1,5 bis 10% der Gesamtheit der Reaktionsmasse miteinander umgesetzt werden, sodann nach und nach unter Rühren der größere Teil des Formaldehyds in fester Polyoxymethylenform derart zugegeben wird, daß die Temperatur zwischen 40 und 58°C gehalten, wird, wobei der pH-Wert des Reaktionsmediums zwischen 7 und 11 liegt, nach der Kondensation das Re-, aktionsprodukt bei einer Temperatur in der Größenordnung von 55°C mit Stearinsäure bis zu einem pH-Wert zwischen 4 und 5 neutralisiert wird und dieses Reaktionsprodukt in kristalliner Form durch Abkühlen oder Rühren unter Mitreißen des Wassers und der flüchtigen Verunreinigungen des rohen Reaktionsmediums mit Hilfe eines Inertgasstromes erhalten wird.